# EUROPÄISCHE PATENTANMELDUNG

(11) **EP 3 498 233 A1**
(43) Veröffentlichungstag der Anmeldung: **19.06.2019**
(21) Anmeldenummer: 17207299.3
(22) Anmeldetag: 14.12.2017
(51) Int. Cl.: A61F 2/958, G01N 3/00, A61F 2/95

(54) **VERFAHREN UND VORRICHTUNG ZUR FESTSTELLUNG EINER AUSREICHENDEN STENTABZUGSKRAFT**

(71) Anmelder: Biotronik AG, 8180 Bülach (CH)
(72) Erfinder: Anderegg, Heinz, 5620 Bremgarten (CH); Rüger, Ilona, 8222 Beringen (CH); Lehner, Beat, 5443 Niederrohrdorf (CH); Vondendriesch, Dennis, 8413 Neftenbach (CH)
(74) Vertreter: Galander, Marcus

(57) **Zusammenfassung**

Die Erfindung betrifft ein Verfahren zur Feststellung einer ausreichenden Stentabzugskraft (F_{A}), aufweisend die Schritte: Bereitstellen eines an einem Schaft (102) festgelegten Ballons (101) mit einem auf dem Ballon (101) festgeklemmten Stent (100), wobei sich Schaft (102), Ballon (101) und Stent (100) entlang einer axialen Richtung (z) erstrecken, [das Festklemmen wird auch als Crimpen bezeichnet], so dass eine Stentabzugskraft (F_{A}) auf den Ballon (101) in der axialen Richtung (z) angewendet werden muss, um den Ballon (101) vom Stent (100) in der axialen Richtung (z) abzuziehen, Einspannen des den Ballon (101) umgebenden Stents (100) zwischen einer ersten und einer zweiten Haltebacke (10, 20) mit einer vordefinierten Anpresskraft (F_{B}) senkrecht zur axialen Richtung (z), Ausüben einer vordefinierten, gewünschten Stentabzugskraft (F_{A}) auf den Ballon (101) über den Schaft (102) in der axialen Richtung (z), bei der der Ballon (101) nicht vom Stent (100) abgezogen werden soll, wobei bei einem Verschieben des Ballons (101) gegenüber dem Stent (100) in der axialen Richtung (z) die Stentabzugskraft (F_{A}) als zu gering angesehen wird und die Schaft-Ballon-Stent Anordnung als Ausschuss verworfen wird, und wobei bei einer konstanten Position des Stents (100) bezüglich des Ballons (101) die Stentabzugskraft (F_{A}) als ausreichend angesehen wird. Des Weiteren betrifft die Erfindung einen entsprechende Vorrichtung (1) zur Feststellung einer ausreichenden Stentabzugskraft (F_{A}).

## Beschreibung

Die vorliegende Erfindung betrifft ein Verfahren und eine Vorrichtung zur Feststellung einer ausreichenden Stentabzugskraft oder Stenthaltekraft nachfolgend auch Implantatabzugskraft genannt. Die Erfindung wird im Folgenden am Beispiel eines ballonexpandierbaren Stents und einem zugehörigen Ballonkatheter beschrieben, ist aber prinzipiell nicht auf diese Anwendung beschränkt sondern generell zur Feststellung der Abzugskraft eines Implantates geeignet.

Stents oder Gefäßstützen kommen im Bereich von Stenosen von Blutgefäßen zur Anwendung, um das jeweilige verengte Gefäße offenzuhalten bzw. den Durchflussquerschnitt zu erweitern. Der jeweilige Stent wird hierzu z.B. auf einen Ballon gecrimpt, der über einen mit dem Ballon verbundenen Schaft aufblasbar ist, so dass der im verengten Gefäßbereich angeordnete Stent auf einen erweiterten Durchmesser expandierbar ist, um die verengte Gefäßstelle offenzuhalten.

Eine wichtige Kenngröße hinsichtlich eines derartigen Stents ist die sogenannte Stent Retention Force (SRF), die hierin auch als Stentabzugskraft bezeichnet wird. Diese Stentabzugskraft bezeichnet eine Kraft, welche auf den Stent ausgeübt werden muss, um diesen axial auf dem Ballon zu verschieben bzw. vom Ballon abzuziehen. Zur Bestimmung dieser Kraft kann z.B. der Stent gehalten werden, wobei auf den Ballon in der axialen Richtung eine Zugkraft ausgeübt wird. Diese kann z.B. über den in axialer Richtung erstreckten Schaft auf den Ballon ausgeübt werden.

Gegenwärtige Methoden zur Bestimmung einer ausreichenden Stentabzugskraft bestimmen diese z.B. wie folgt. Gemäß einer ersten Variante wird die Stent-Ballon-Kombination im Zentrum einer U-förmigen Halterung mit zwei Klebestreifen fixiert.

Die Halterung wird mit einer bestimmten Kraft gezogen, während der Schaft (Ballon) am anderen Ende gehalten wird. Wird mit der gewünschten Stentabzugskraft an der Halterung gezogen, muss der Stent auf dem Ballon verbleiben bzw. darf sich auf diesem nicht axial verschieben. Andernfalls gilt der Test als nicht bestanden.

Alternativ hierzu wird gemäß einem weiteren Testverfahren die Ballon-Stent-Kombination in der Crimpvorrichtung belassen und am Schaft mit vorgegebener Kraft gezogen.

Des Weiteren besteht die Möglichkeit, mit einem hakenförmigen Werkzeug an den Stentstreben anzugreifen, um den Stent vom Ballon abzuziehen.

Nachteilig an den vorgenannten Methoden ist insbesondere der Umstand, dass die Fixierung der Stent-Ballon-Kombination in der vorgenannten Halterung manuell vorgenommen werden muss und daher aufwändig ist. Ferner können die besagten Klebestreifen nicht nur am Stent angreifen, sondern auch am darunter angeordneten Ballon, was die Fehleranfälligkeit des Verfahrens entsprechend erhöht.

Der vorliegenden Erfindung liegt hiervon ausgehend die Aufgabe zugrunde, ein verbessertes Verfahren und eine verbesserte Vorrichtung zur Feststellung einer ausreichenden Stentabzugskraft bereitzustellen.

Dieses Problem wird durch eine Vorrichtung mit den Merkmalen des Anspruchs 8 sowie durch ein Verfahren mit den Merkmalen des Anspruchs 1 gelöst. Vorteilhafte Ausgestaltungen des jeweiligen Erfindungsaspekts sind in den entsprechenden Unteransprüchen angegeben und werden nachfolgend beschrieben.

Gemäß Anspruch 1 wird ein Verfahren zur Feststellung einer ausreichenden Abzugskraft offenbart, aufweisend die Schritte:
- Bereitstellen eines an einem Katheterteil festgeklemmten Implantats, (100), wobei sich Katheterteil und Implantat entlang einer axialen Richtung (z) erstrecken, so dass eine Abzugskraft (F_{A}) auf den Katheterteil in der axialen Richtung (z) überschritten werden muss, um den Katheterteil bezüglich des Implantats in der axialen Richtung (z) zu verschieben,
- Einspannen des auf dem Katheterteil festgeklemmten Implantats zwischen einer ersten und einer zweiten Haltebacke (10, 20) mit einer vordefinierten Anpresskraft (F_{B}) senkrecht zur axialen Richtung (z),
- Ausüben einer vordefinierten, gewünschten Abzugskraft (F_{A}) auf den Katheterteil in der axialen Richtung (z), bei der der Katheterteil sich nicht bezüglich des Implantats in der axialen Richtung (z) verschieben soll, wobei bei einem Verschieben des Katheterteils gegenüber dem Implantat in der axialen Richtung (z) die Abzugskraft (F_{A}) des Implantats als zu gering angesehen wird, und wobei bei einer konstanten Position des Implantats bezüglich des Katheterteils die Abzugskraft (F_{A}) des Implantats (100) als ausreichend angesehen wird

Im Folgenden wird die Erfindung anhand des Beispiels eines Verfahrens zur Feststellung der Stentabzugskraft für einen Stent beschrieben, welcher auf einem Ballon festgeklemmt ist, der an einem Katheterschaft festgelegt ist. Entsprechend wird alles, was im Folgenden für einen an einem Schaft festgelegt Ballon und einem darauf festgeklemmten Stent erörtert wird, als synonym für ein Katheterteil und ein darauf festgeklemmtes Implantat verstanden. Die Erfindung ist jedoch insbesondere für die Feststellung der Stentabzugskraft für einen auf einem Ballon festgeklemmten Stent geeignet.

Als ein Verschieben des Ballons bezüglich des Stents beim Ausüben der besagten vordefinieren, gewünschten Stentabzugskraft wird dabei auch ein vollständiges Abziehen des Ballons vom Stent betrachtet.

Weiterhin ist gemäß einer Ausführungsform des erfindungsgemäßen Verfahrens vorgesehen, dass die Haltebacken lediglich den Stent kontaktieren bzw. halten. Diesbezüglich ist insbesondere vorgesehen, dass die beiden Haltebacken lediglich eine Außenseite des Stents kontaktieren bzw. halten, die in einer senkrecht zur axialen Richtung verlaufenden Umfangsrichtung den Ballon umläuft. Die Haltebacken halten also insbesondere nicht unmittelbar den Ballon oder den vom Ballon abgehenden Schaft.

Weiterhin ist gemäß einer Ausführungsform der Erfindung vorgesehen, dass die beiden Haltebacken mit je einer Oberfläche mit der Anpresskraft gegen den Stent gepresst werden, wobei jene Oberfläche aus einem der folgenden Stoffe gebildet ist: Silikon, Polyurethan oder sonstige Elastomere.

Gemäß einer weiteren Ausführungsform des erfindungsgemäßen Verfahrens ist vorgesehen, dass die erste Haltebacke mittels einer vorgespannten Feder zum Ausüben der Anpresskraft gegen den Stent gepresst wird, so dass die erste Haltebacke den Stent gegen die zweite Haltebacke drückt und so der den Stent umgreifende Ballon zwischen den beiden Haltebacken mit der Anpresskraft eingespannt wird.

Weiterhin ist bei dem Verfahren gemäß einer Ausführungsform vorgesehen, dass die axiale Richtung vertikal verläuft, wobei insbesondere die auf den Ballon ausgeübte Stentabzugskraft in vertikaler Richtung nach unten weist.

Ein weiterer Aspekt der vorliegenden Erfindung betrifft die Bereitstellung einer Messnormal-Einrichtung, die ein Messnormal definiert. Diese Einrichtung kann dazu verwendet werden, eine genau vordefinierbare Zugkraft auf den Ballon auszuüben.

Diese Messnormal-Einrichtung kann zwischen ein freies Ende des Schaftes und einer Befestigungsvorrichtung eingefügt werden, wobei über die Befestigungsvorrichtung in der axialen Richtung an der Messnormal-Einrichtung gezogen wird. Diese Zugkraft wird des Weiteren über die Messnormal-Einrichtung in das freie Ende des Schafts und somit in den Ballon eingeleitet.

Die Messnormal-Einrichtung weist gemäß einer Ausführungsform einen ersten Abschnitt und einen damit verbundenen zweiten Abschnitt auf, wobei die beiden Abschnitte sich voneinander lösen, wenn sie mit einer vordefinierten Kraft auseinander gezogen werden. Auf diese Weise kann eine vordefinierte Zugkraft auf den Schaft/Ballon in der axialen Richtung ausgeübt werden, da sich die beiden Abschnitte beim Erreichen dieser Kraft voneinander trennen.

Gemäß einer Ausführungsform können die beiden Abschnitte jeweils durch einen Magneten gebildet sein, wobei sich die beiden Magnete mit einer vordefinierbaren Kraft anziehen. Die zur Trennung der beiden Abschnitte / Magnete nötige Kraft kann z.B. durch entsprechende Wahl der Magnete eingestellt werden. Die Messnormal-Einrichtung kann auch mehr als zwei Magnete aufweisen.

Bei Verwendung der Messnormal-Einrichtung kann in beliebiger Weise (d.h. manuell oder maschinell) in der axialen Richtung an der Messnormal-Einrichtung gezogen werden. Die Zugkraft wird dabei so ausgeübt bzw. gesteigert, dass sich die Messnormal-Einrichtung öffnet, d.h., die beiden Abschnitte der Messnormal-Einrichtung werden voneinander gelöst. Für den Fall, dass die zur Öffnung der Messnormal-Einrichtung notwendige Kraft der zu prüfenden bzw. gewünschten Stentabzugskraft entspricht, kann festgestellt werden, ob der Stent eine ausreichende Stentabzugskraft aufweist. Für den Fall, dass sich der Stent axial bewegt bzw. vom Ballon abgezogen wird, bevor sich die Messnormal-Einrichtung öffnet (d.h. die beiden Abschnitte der Messnormal-Einrichtung voneinander gelöst werden), ist die Stentabzugskraft nicht ausreichend und die Ballon-Stent-Kombination wird als Ausschuss verworfen.

Ein weiterer Aspekt der vorliegenden Erfindung betrifft eine Vorrichtung zur Feststellung einer ausreichenden Stentabzugskraft. Diese Vorrichtung kann insbesondere zur Durchführung des erfindungsgemäßen Verfahrens verwendet werden.

Die erfindungsgemäße Vorrichtung weist dabei zumindest auf:
- eine Einspannvorrichtung aufweisend eine erste und eine zweite Haltebacke, wobei die beiden Haltebacken einander gegenüber liegen und zum Halten eines einen Ballon umgebenden Stents mit einer vordefinierbaren Anpresskraft konfiguriert sind, und
- einen Aktuator zum Ausüben einer vordefinierbaren Zugkraft auf einen an dem Ballon festgelegten Schaft, wobei der Aktuator eine Befestigungsvorrichtung zum Befestigen des Schaftes bezüglich des Aktuators aufweist, so dass die Zugkraft über die Befestigungsvorrichtung auf den Schaft und somit den Ballon ausübbar ist.

Gemäß einer Ausführungsform der erfindungsgemäßen Vorrichtung ist vorgesehen, dass die erste Haltebacke einen ersten Materialbereich und die zweite Haltebacke einen zweiten Materialbereich aufweist, wobei die beiden Materialbereiche zum Kontaktieren und Halten des Stents jeweils eine Oberfläche aufweisen, wobei die beide Oberflächen parallel zueinander sowie senkrecht zur Anpresskraft verlaufen.

Die beiden Materialbereiche können z.B. als hexaedrische Kissen ausgebildet sein, andere Formen sind auch denkbar.

Weiterhin ist gemäß einer bevorzugten Ausführungsform der Erfindung vorgesehen, dass die beiden Materialbereiche jeweils aus einem der folgenden Stoffe gebildet sind: einem Silikon (d.h. einem Stoff aus der Gruppe der Poly(organo)siloxane) oder PUR.

Weiterhin ist gemäß einer Ausführungsform der Vorrichtung vorgesehen, dass die erste Haltebacke einen ersten Träger aufweist und dass die zweite Haltebacke einen zweiten Träger aufweist, wobei die beiden Träger einander gegenüberliegen, und wobei der erste Materialbereich an dem ersten Träger und der zweite Materialbereich an dem zweiten Träger festgelegt ist.

Gemäß einer weiteren Ausführungsform der Erfindung ist vorgesehen, dass der erste Träger an einem ersten Arm der Einspannvorrichtung festgelegt ist, und dass der zweite Träger an einem zweiten Arm der Einspannvorrichtung festgelegt ist.

Hierbei ist gemäß einer Ausführungsform bevorzugt vorgesehen, dass der erste Träger zur Erzeugung der Anpresskraft mittels einer Feder in Richtung auf den zweiten Träger vorspannbar ist.

Weiterhin ist gemäß einer Ausführungsform der Vorrichtung vorgesehen, dass sich die Feder zum Einstellen der Anpresskraft an einer Schraube abstützt, die mit einem Außengewinde in ein Innengewinde des ersten Armes eingreift. Die Feder ist hierbei vorzugsweise zwischen dem ersten Träger und der Schraube angeordnet. Durch zunehmendes Einschrauben der Schraube in das Innengewinde ist die Vorspannung der Feder bzw. die Anpresskraft einstellbar. Hier kommen aber auch alternative Möglichkeiten zur Krafteinstellung wie beispielsweise eine Pneumatik in Frage.

Weitere Merkmale und Ausführungsformen der Erfindung werden nachfolgend anhand der Figuren erläutert. Es zeigen:
- Fig. 1: eine Schnittdarstellung einer Einspannvorrichtung einer erfindungsgemäßen Vorrichtung zum Halten des den Ballon umgebenden Stents; und
- Fig. 2: eine Schnittdarstellung einer Befestigungsvorrichtung zum Einleiten einer Zugkraft in den mit dem Ballon verbundenen Schaft.

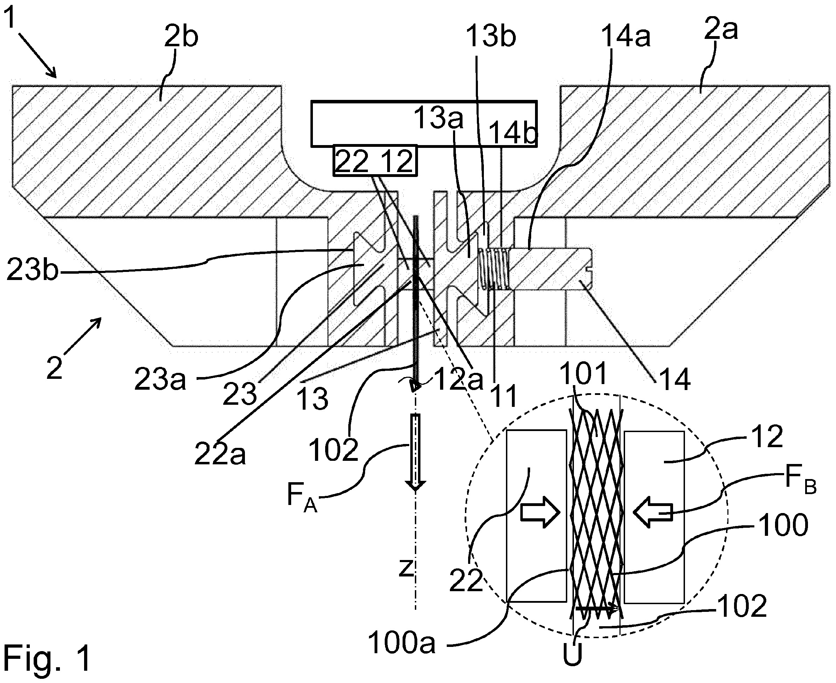

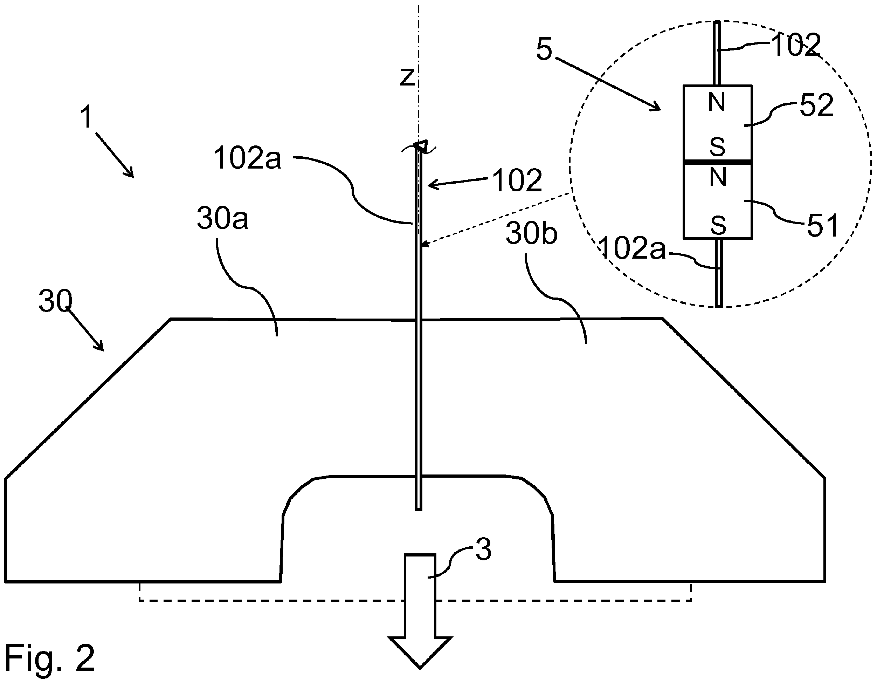

Die Figur 1 zeigt im Zusammenhang mit der Figur 2 eine erfindungsgemäße Vorrichtung 1 zum Testen der Stentabzugskraft einer Ballon-Stent-Kombination 101, 100. Hierbei ist der Stent 100 auf einem Ballon 101 festgeklemmt, so dass der Stent 100 den Ballon 101 in einer Umfangsrichtung U (also quer zur axialen Richtung z des Stents 100 bzw. des Ballons 101) umgreift. Vom Ballon 101 geht in der axialen Richtung z ein Schaft 102 ab, über den der Ballon 101 aufblasbar ist. Die Figur 1 zeigt vorliegend einen oberen Teil der Vorrichtung 1, nämlich eine Einspannvorrichtung 2 für den Stent 100. Ein unterer Teil der Vorrichtung 2, der zum Ausüben einer Zugkraft F_{A} auf den Ballon 101 konfiguriert ist, die in axialer bzw. vertikaler Richtung z nach unten weist, ist in der Figur 2 dargestellt.

Um den Stent 100 vom Ballon 101 bzw. umgekehrt den Ballon 101 vom Stent 100 abzuziehen bzw. die beiden Komponenten 100, 101 axial zueinander zu verschieben, muss die sogenannte Stentabzugskraft F_{A} in der axialen Richtung z aufgewendet werden, die hinreichend groß dimensioniert sein muss, damit die Funktionalität des Ballonkatheters (Stent-Ballon-Kombination 100, 101) sichergestellt werden kann.

Ob eine ausreichend große Stentabzugskraft F_{A} vorliegt, kann erfindungsgemäß wie folgt getestet werden. Zunächst wird der den Ballon 101 umgebende Stent 100 zwischen einer ersten und einer zweiten Haltebacke 10, 20 mit einer vordefinierten Anpresskraft F_{B} (beispielsweise zwischen 1 und 10 N) senkrecht zur axialen Richtung z eingespannt. Hierbei ist bevorzugt vorgesehen, dass die beiden Haltebacken 10, 20 mit je einer Oberfläche 12a, 22a lediglich eine Außenseite 100a des Stents 100 kontaktieren bzw. halten, die in der senkrecht zur axialen Richtung z verlaufenden Umfangsrichtung U den Ballon 101 umläuft und dem Ballon 101 abgewandt ist. Die Oberflächen 12a, 22a sind vorzugsweise aus Silikon oder PUR oder einem vergleichbaren Material gebildet.

Ein freies Ende 102a des Schaftes 102 wird gemäß Figur 2 vorzugsweise in einer Befestigungsvorrichtung 30 eines Aktuators 3 eingespannt. Über die Befestigungsvorrichtung 30 wird dann mittels des Aktuators 3 eine vordefinierte, gewünschten Stentabzugskraft F_{A} (beispielsweise zwischen 1 und 15 N, insbesondere zwischen 10 und 15 N) auf den Ballon 101 über den Schaft 102 in der axialen Richtung z ausgeübt. Verschiebt sich nun der Ballon 101 gegenüber dem Stent 100 in der axialen Richtung z, wird die Stentabzugskraft F_{A} des Stents 100 als zu gering angesehen. Andernfalls gilt der Test als bestanden.

Gemäß Figur 1 ist hinsichtlich der Einspannvorrichtung 2 vorzugsweise vorgesehen, dass die erste Haltebacke 10 einen ersten Materialbereich 12 und die zweite Haltebacke 20 einen zweiten Materialbereich 22 aufweist, wobei die beiden Materialbereiche 12, 22 vorzugsweise als Kissen ausgebildet sind und die besagten Oberflächen 12a, 22a zum Halten des Stents 100 ausbilden. Die beiden Oberflächen 12a, 22a verlaufen parallel zueinander sowie senkrecht zur Anpresskraft F_{B}. Die besagten Materialbereiche/Kissen 12, 22 sind vorzugsweise, wie oben bereits dargelegt, aus einem Silikon oder aus PUR gefertigt.

Zum Halten der beiden Materialbereiche 12, 22 weisen die beiden Haltebacken 10, 20 vorzugsweise je einen Träger 13, 23 auf, wobei die beiden Träger 13, 23 einander gegenüberliegen, und wobei der erste Materialbereich 12 an dem ersten Träger 13 und der zweite Materialbereich 22 an dem zweiten Träger (23) festgelegt ist. Hierbei ist der erste Träger 13 an einem ersten Arm 2a der Einspannvorrichtung befestigt, wobei insbesondere der erste Träger 13 mit einem Vorsprung 13a in eine Ausnehmung 13b des ersten Armes 2a eingreift. Weiterhin ist vorgesehen, dass der erste Träger 13 zur Erzeugung der Anpresskraft F_{B} mittels einer Feder 11 in Richtung auf den zweiten Träger 23 vorspannbar ist, wobei sich die Feder 11 zum Einstellen der Anpresskraft F_{B} an einer Schraube 14 abstützt, die mit einem Außengewinde 14a in ein Innengewinde 14b des ersten Armes 2a eingreift. Die Feder 11 ist hierbei zwischen dem ersten Träger 13 und der Schraube 14 angeordnet. Weiterhin kann auch der zweite Träger 23 an dem zweiten Arm 2b der Einspannvorrichtung 2 festgelegt sein, indem der zweite Träger 23 mit einem Vorsprung 23a in eine Ausnehmung 23b des zweiten Armes 2b eingreift.

Gemäß Figur 2 kann des Weiteren die oben bereits erwähnte Befestigungsvorrichtung 30 zwei Klemmbacken 30a, 30b zum Festklemmen eines freien Endes 102a des Schaftes 2 aufweisen, so dass über die Befestigungsvorrichtung 30 die besagte Zugkraft bzw. vordefinierte, gewünschte Stentabzugskraft F_{A} auf den Ballon 101 ausübbar ist. Die Zugkraft F_{A} kann z.B. mittels eines Aktuators 3 über die Befestigungsvorrichtung 30 auf den Schaft 102 in der axialen Richtung z ausgeübt werden.

Figur 2 zeigt des Weiteren eine Messnormal-Einrichtung 5, die dazu verwendet werden kann, eine genau vordefinierbare Zugkraft auf den Ballon 101 auszuüben, insbesondere für den Fall, dass die Zugkraft F_{A} nicht genau steuerbar/messbar ist, z.B. bei einem manuellen Ausüben der Zugkraft F_{A}.

Die Messnormal-Einrichtung 5 kann gemäß Figur 2 optional zwischen ein freies Ende 102a des Schaftes 102a und der Befestigungsvorrichtung 30 eingefügt werden, wobei über die Befestigungsvorrichtung 30 in der axialen Richtung z an der Messnormal-Einrichtung 5 gezogen wird. Diese Zugkraft F_{A} wird des Weiteren über die Messnormal-Einrichtung 5 in das freie Ende 102a des Schafts 102 und somit in den Ballon 101 eingeleitet.

Die Messnormal-Einrichtung 5 kann z.B. einen ersten Abschnitt 51 und einen damit verbundenen zweiten Abschnitt 52 aufweisen, wobei die beiden Abschnitte 51, 52 sich voneinander lösen, wenn sie mit einer vordefinierten Kraft auseinander gezogen werden. Auf diese Weise kann eine vordefinierte Zugkraft F_{A} auf den Schaft 102 bzw. Ballon 101 in der axialen Richtung z ausgeübt werden, da sich die beiden Abschnitte 51, 52 beim Erreichen dieser Kraft F_{A} voneinander trennen und die ausgeübte Kraft F_{A} genau begrenzen. Die beiden Abschnitte 51, 52 können z.B. jeweils durch einen Magneten 51, 52 gebildet sein, wobei sich die beiden Magnete 51, 52 mit der vordefinierbaren Kraft F_{A} anziehen, die z.B. durch die Wahl der Magnete einstellbar ist.

## Patentansprüche

1. Verfahren zur Feststellung einer ausreichenden Abzugskraft (F_{A}), aufweisend die Schritte:
- Bereitstellen eines an einem Katheterteil festgeklemmten Implantats, (100), wobei sich Katheterteil und Implantat entlang einer axialen Richtung (z) erstrecken, so dass eine Abzugskraft (F_{A}) auf den Katheterteil in der axialen Richtung (z) überschritten werden muss, um den Katheterteil bezüglich des Implantats in der axialen Richtung (z) zu verschieben,
- Einspannen des auf dem Katheterteil festgeklemmten Implantats zwischen einer ersten und einer zweiten Haltebacke (10, 20) mit einer vordefinierten Anpresskraft (F_{B}) senkrecht zur axialen Richtung (z),
- Ausüben einer vordefinierten, gewünschten Abzugskraft (F_{A}) auf den Katheterteil in der axialen Richtung (z), bei der der Katheterteil sich nicht bezüglich des Implantats in der axialen Richtung (z) verschieben soll, wobei bei einem Verschieben des Katheterteils gegenüber dem Implantat in der axialen Richtung (z) die Abzugskraft (F_{A}) des Implantats als zu gering angesehen wird, und wobei bei einer konstanten Position des Implantats bezüglich des Katheterteils die Abzugskraft (F_{A}) des Implantats (100) als ausreichend angesehen wird.

2. Verfahren nach Anspruch 1, **dadurch gekennzeichnet, dass** der Katheterteil aus einem Schaft (102) und einem daran festgelegten Ballon (101) besteht und das Implantat ein Stent (100) und die Abzugskraft eine Stentabzugskraft ist.

3. Verfahren nach Anspruch 1, **dadurch gekennzeichnet, dass** die Haltebacken (10, 20) lediglich das Implantat, insbesondere den Stent (100), kontaktieren.

4. Verfahren nach Anspruch 1 oder 2, **dadurch gekennzeichnet, dass** die beiden Haltebacken (10, 20) lediglich eine Außenseite (100a) des Implantats, insbesondere des Stents (100), kontaktieren, die in einer senkrecht zur axialen Richtung (z) verlaufenden Umfangsrichtung (U) den Katheterteil, insbesondere den Ballon (101), umläuft.

5. Verfahren nach einem der vorhergehenden Ansprüche, **dadurch gekennzeichnet, dass** die beiden Haltebacken (10, 20) mit je einer Oberfläche (12a, 22a) gegen das Implantat, insbesondere den Stent (100), gepresst werden, wobei jene Oberfläche (12a, 22a) aus einem der folgenden Stoffe gebildet ist: Silikon, PUR.

6. Verfahren nach einem der vorhergehenden Ansprüche, **dadurch gekennzeichnet, dass** zum Ausüben der Anpresskraft (F_{B}) die erste Haltebacke (10, 20) mittels einer vorgespannten Feder (11) gegen das Implantat, insbesondere den Stent (100), gepresst wird, so dass die erste Haltebacke (10) den Stent (100) gegen die zweite Haltebacke (20) drückt und so das den Katheterteil, insbesondere den Ballon (101), umgreifende Implantat, insbesondere der Stent (100), zwischen den beiden Haltebacken (10, 20) mit der Anpresskraft (F_{B}) eingespannt wird.

7. Verfahren nach einem der vorhergehenden Ansprüche, **dadurch gekennzeichnet, dass** die axiale Richtung (z) vertikal verläuft, wobei insbesondere die auf den Katheterteil, insbesondere den Ballon (101) ausgeübte Abzugskraft, insbesondere Stentabzugskraft (F_{A}), in vertikaler Richtung (z) nach unten weist.

8. Vorrichtung (1) zur Feststellung einer ausreichenden Abzugskraft (F_{A}), mit
- einer Einspannvorrichtung (2) aufweisend eine erste und eine zweite Haltebacke (10, 20), wobei die beiden Haltebacken (10, 20) einander gegenüber liegen und zum Halten eines einen Katheterteil umgebenden Implantat, insbesondere einen Ballon (101) umgebenden Stents (100), mit einer vordefinierbaren Anpresskraft (F_{B}) konfiguriert sind, und
- einem Aktuator (3) zum Ausüben einer vordefinierbaren Zugkraft (F_{A}) auf den Katheterteil, insbesondere auf einen an dem Ballon (101) festgelegten Schaft, (102), wobei der Aktuator (3) eine Befestigungsvorrichtung (30) zum Befestigen des Katheterteils, insbesondere des Schaftes (102), bezüglich des Aktuators (3) aufweist, so dass die Zugkraft (F_{A}) über die Befestigungsvorrichtung (30) auf den Katheterteil, insbesondere auf den Schaft (102) und somit den Ballon (101), ausübbar ist.

9. Vorrichtung nach Anspruch 8, **dadurch gekennzeichnet, dass** die erste Haltebacke (10) einen ersten Materialbereich (12) und die zweite Haltebacke (20) einen zweiten Materialbereich (22) aufweist, wobei die beiden Materialbereiche (12, 22) zum Kontaktieren und Halten des Implantats, insbesondere des Stents (100), jeweils eine Oberfläche (12a, 22a) aufweisen, wobei die beide Oberflächen (12a, 22a) parallel zueinander sowie senkrecht zur Anpresskraft (F_{B}) verlaufen.

10. Vorrichtung nach Anspruch 9, **dadurch gekennzeichnet, dass** die beiden Materialbereiche (12, 22) jeweils aus einem der folgenden Stoffe gebildet sind: einem Silikon, PUR.

11. Vorrichtung nach Anspruch 7 oder 8, **dadurch gekennzeichnet, dass** die erste Haltebacke (10) einen ersten Träger (13) aufweist, und dass die zweite Haltebacke (20) einen zweiten Träger (23) aufweist, wobei die beiden Träger (13, 23) einander gegenüberliegen, und wobei der erste Materialbereich (12) an dem ersten Träger (13) und der zweite Materialbereich (22) an dem zweiten Träger (23) festgelegt ist.

12. Vorrichtung nach Anspruch 11, **dadurch gekennzeichnet, dass** der erste Träger (13) an einem ersten Arm (2a) der Einspannvorrichtung (2) festgelegt ist, und dass der zweite Träger (23) an einem zweiten Arm (2b) der Einspannvorrichtung (2) festgelegt ist.

13. Vorrichtung nach Anspruch 11 oder 12, **dadurch gekennzeichnet, dass** der erste Träger (13) zur Erzeugung der Anpresskraft (F_{B}) mittels einer Feder (11) in Richtung auf den zweiten Träger (23) vorspannbar ist.

14. Vorrichtung nach Anspruch 13, **dadurch gekennzeichnet, dass** sich die Feder (11) zum Einstellen der Anpresskraft (FB) an einer Schraube (14) abstützt, die mit einem Außengewinde (14a) in ein Innengewinde (14b) des ersten Armes (2a) eingreift.
